(19)

Europäisches Patentamt
European Patent Office
Office européen des brevets

(11) Numéro de publication: **0 501 031 B1**

(12) **FASCICULE DE BREVET EUROPEEN**

(45) Date de publication du fascicule du brevet: **06.09.95**

(51) Int. Cl.[6]: **C07C 251/16**, A61K 7/46, C07C 229/56

(21) Numéro de dépôt: **91121412.0**

(22) Date de dépôt: **13.12.91**

(54) **Ingrédient parfumant.**

(30) Priorité: **27.02.91 CH 599/91**
**25.10.91 CH 3138/91**

(43) Date de publication de la demande:
**02.09.92 Bulletin 92/36**

(45) Mention de la délivrance du brevet:
**06.09.95 Bulletin 95/36**

(84) Etats contractants désignés:
**CH DE FR GB LI NL**

(56) Documents cités:
**US-A- 4 775 720**

(73) Titulaire: **FIRMENICH SA**
**1, route des Jeunes**
**CH-1211 Genève 8 (CH)**

(72) Inventeur: **Blanc, Pierre-Alain**
**Chemin du Levrioux**
**CH-1263 Crassier (CH)**
Inventeur: **Aschiero, Roland**
**101, chemin de Saule**
**CH-1233 Bernex (CH)**

(74) Mandataire: **Salvadori, Giuseppe, Dr.**
**c/o Firmenich S.A.**
**Case Postale 239**
**CH-1211 Genève 8 (CH)**

**EP 0 501 031 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a trait au domaine de la parfumerie. Elle concerne plus particulièrement un nouvel ingrédient parfumant appartenant au groupe de produits généralement connus sous la désignation de bases de Schiff.

Les bases de Schiff sont bien connues dans l'art de la parfumerie. C'est ainsi que l'oeuvre de référence de S. Arctander, Perfume and Flavor Chemicals, Montclair, N.J., USA (1969), cite un grand nombre de ces bases [voir, par exemple, sects 26, 157, 244, 624, 655, 665, 755, 759, 836, 1549, 1621, 1735, 1782, 1799, 1955, 2080, 2125, 2135, 2253, 2280 ou 3070]. Les caractères odorants de ces bases de Schiff se ressemblent souvent. En effet, une grande majorité de ces produits sont dits posséder des odeurs florales, fruitées, notamment de type citrus et, plus particulièrement, réminiscentes de l'odeur de la fleur d'oranger.

D'autres bases de Schiff ont été décrites, plus récemment, dans les brevets US 4,775,720, 4,839,083 et 4,840,801 ainsi que JP 62-153212. Cependant, malgré le nombre de produits de ce type décrits à ce jour, nous n'avons trouvé aucune mention du produit de l'invention dans l'art antérieur.

La présente invention a pour objet le produit obtenu par la réaction de l'anthranilate de méthyle avec le TRIFERNAL ® (3-phénylbutanal; origine : Firmenich SA, Genève, Suisse).

Nous avons maintenant découvert que ce produit de réaction possède des propriétés odorantes très originales et tout à fait inattendues au vu de l'art antérieur. En effet, il développe une odeur de type floral-fleur jaune, avec une note verte plus prononcée en tête qu'en fond. Il s'agit d'une note odorante très naturelle qui rappelle l'odeur de la flore alpine au printemps et qui est très originale par rapport aux notes odorantes des bases de Schiff connues. Ceci ressort clairement du tableau présenté ci-après qui décrit les résultats de l'évaluation olfactive comparative, effectuée par un panel d'experts parfumeurs, entre le produit faisant l'objet de la présente invention et plusieurs bases de Schiff choisies parmi celles d'usage plus courant en parfumerie.

## TABLEAU

TABLEAU

| Composé | Source descriptive/origine | Caractères odorants: Fleur d'oranger | Néroli | Petitgrain | Vert | Fleur blanche | Poudré | Fleur jaune | Autres |
|---|---|---|---|---|---|---|---|---|---|
| | Firmenich SA | +++ | ++ | + | + | | | | Aldéhydé |
| | Arctander 1735 | +++ | ++ | | | ++ | | | Muguet |
| | Arctander 2080 | ++ | ++ | | | ++ | | | |
| | Arctander 1799 | + | | | ++ | | ++ | | Cyclamen /aqueux |
| | Firmenich SA | + | | ++ | +++ | | | | |
| | US 4,840,801 JP 62-153212 | | + | | | | +++ | | Anisique/ cassie |
| | US 4,839,083 JP 62-153212 | ++ | | | | ++ | ++ | | |
| Produit selon la présente invention | Voir exemples | | | | ++ | | + | +++ | Mimosa |

Ordre décroissant d'intensité +++ → ++ → +

Ce tableau montre que, contrairement à ce que l'on observe avec les bases de Schiff connues citées, le produit de réaction selon la présente invention ne possède aucun caractère odorant floral de type hespéridé et qu'il est totalement dépourvu de la note fleur d'oranger si commune parmi les autres bases de Schiff. Par ailleurs, sa note dominante, à savoir de type fleur jaune, est très surprenante car aucune des autres bases de Schiff ne possède ce caractère odorant.

En effet, nous n'avons trouvé dans l'art antérieur aucune base de Schiff possédant la combinaison de propriétés odorantes caractéristiques du produit de réaction de l'invention. La note florale de ce dernier est nettement plus prononcée que celle des bases de Schiff connues, dont les odeurs sont plutôt caractérisées par des notes florales-fruitées. D'autre part, le caractère dominant original de type fleur jaune du produit de l'invention est particulièrement utile, car il rend ce produit d'un usage plus facile en parfumerie que son aldéhyde parent, le 3-phénylbutanal, dont il rappelle l'odeur, mais adoucie.

De par ses propriétés odorantes, le produit de réaction selon l'invention se prête aussi bien à des applications en parfumerie fine qu'en parfumerie fonctionnelle. Il peut être utilisé pour la préparation de compositions parfumantes et articles parfumés de type varié. Il convient très bien à la préparation de parfums et eaux de toilette, au parfumage de savons, de gels de douche ou bain, ainsi que de shampoings, produits cosmétiques ou désodorisants corporels ou d'air ambiant.

Comme il ressort des exemples présentés plus loin, l'excellente substantivité de sa note odorante rend son usage dans le parfumage de détergents et adoucissants textiles également très avantageux et il convient aussi au parfumage de produits d'entretien.

Pour ces applications, il peut être utilisé soit seul, soit, comme il est plus courant en parfumerie, en mélange avec d'autres ingrédients parfumants, des solvants ou des adjuvants usuels. On trouvera des exemples de ces derniers dans les ouvrages de référence dans l'art, tels que, par exemple, le livre d'Arctander précédemment cité.

Les concentrations dans lesquelles le produit de l'invention peut être utilisé varient dans une gamme de valeurs très étendue. Il est bien connu que ces valeurs dépendent de l'effet parfumant recherché et de la nature de l'article que l'on désire parfumer. A titre d'exemple, on peut citer des concentrations de l'ordre de 1 à 10%, voire plus, en poids de composé, par rapport au poids de la composition parfumante dans laquelle il est incorporé. Des concentrations bien inférieures à ces valeurs peuvent être cependant utilisées, notamment lors de l'application du produit de réaction de l'invention dans le parfumage des articles cités auparavant.

Le produit de la réaction de l'anthranilate de méthyle avec le TRIFERNAL ® peut être préparé selon les méthodes usuelles de synthèse des bases de Schiff, en faisant réagir les deux produits de départ. Il est connu [voir, par exemple, le brevet US 4,839,083 déjà cité] que, suivant la nature desdits produits de départ, leur produit de réaction peut être constitué par un mélange de plusieurs isomères de liaison et/ou de configuration.

Dans le cas présent, nous avons constaté que la composition du produit de réaction obtenu dépendait également des conditions de réaction, dont des exemples sont présentés ci-après, et en particulier de la température. Le produit de réaction obtenu pouvait ainsi contenir des quantités variables des composés de formule :

(Ia)

ou

(Ib)

dans laquelle la ligne ondulée représente une liaison de conformation cis ou trans, ou encore de formule

(Ic)

Du point de vue olfactif, tous ces mélanges possédaient des propriétés similaires et pouvaient être utilisés en parfumerie de façon interchangeable pour obtenir des effets odorants comparables. Si désiré, les composants individuels pouvaient être séparés à l'aide des techniques usuelles et leurs données analytiques individuelles sont présentées plus loin.

Description des dessins

Les figs. 1 et 2 représentent le spectre de résonance magnétique nucléaire du proton, pris à 360 MHz dans le $CHCl_3$, du produit de l'invention obtenu, respectivement, selon les méthodes de préparation 1 et 2 -voir ci-après-.

Les exemples de préparation suivants, dans lesquels les températures sont indiquées en degrés centigrades et les abréviations ont le sens usuel dans l'art, décrivent des produits selon l'invention obtenus selon différentes méthodes de préparation.

Méthode 1

Dans un ballon à 3 cols, muni d'un agitateur magnétique et d'un séparateur d'eau, on a chargé 90,6 g (0,60 mole) d'anthranilate de méthyle, 74,0 g (0,50 mole) de 3-phénylbutanal et 300 ml de toluène. On a porté le mélange à reflux pendant 20 h, jusqu'à formation d'une quantité d'eau correspondant à un rendement théorique de 100% (9,0 ml). On a ensuite remplacé le séparateur d'eau par une tête de colonne très courte et distillé le toluène à pression atmosphérique. Après refroidissement, on a appliqué progressivement un vide de $15x12^2$ Pa et distillé l'excès d'anthranilate de méthyle (temp. maximale du bain d'huile : 110°). On a refroidi, placé sous vide poussé et distillé le produit de la réaction (temp. bain : 110°/0,1 Pa) assez rapidement et en contrôlant la température soigneusement pour éviter des décompositions.
La figure I montre le spectre RMN du proton, pris à 360 MHz dans $CHCl_3$, du produit de réaction obtenu.
Les spectres de couplage CG/SM (colonne capillaire SPB1 30 m, 160°-230°, 7 min) des composants du mélange étaient les suivants :

A. N-(3-phénylbutylidène)anthranilate de méthyle
(10% en poids du mélange)
SM : 281($M^+$,20), 266(6), 234(43), 131(51), 117(55), 105(100), 77(98)

B. (E)-N-(3-phényl-1-buténył)anthranilate de méthyle
(30% en poids du mélange)
SM : 281($M^+$,37), 266(59), 234(73), 117(100), 105(78), 77(85)

C. (Z)-N-(3-phényl-1-buténył)anthranilate de méthyle
(60% en poids du mélange)
SM : 281($M^+$,42), 266(71), 234(84), 116(80), 105(84), 77(100)

Méthode 2

Dans un ballon à 3 cols muni d'un agitateur magnétique et d'un séparateur d'eau, on a chargé, à 20° et sous vigoureuse agitation, 90,6 g (0,60 mole) d'anthranilate de méthyle. On a ajouté 5 g d'un total de 74,0 g (0,50 mole) de 3-phénylbutanal. La réaction était exothermique et la température est montée à 25°. On a chauffé à 45° et introduit le restant de l'aldéhyde en 1 h. A cette même température, on a appliqué progressivement un vide de 10 mm ($13x10^2$ Pa) et, ensuite, on a chauffé 2 h à 60° pour éliminer par distillation une partie de l'eau (4,85 g) formée au cours de la réaction. Les produits de départ non réagis ont été distillés (temp. bain : 110°/0,1 Pa) de la même manière.
Le spectre RMN du proton, obtenu à 360MHz dans $CDCl_3$, effectué sur le résidu (environ 140 g)

précédemment obtenu est représenté à la figure 2. Il confirme la présence dans le mélange des trois composés A (10% en poids), B (30% en poids) et C (35% en poids) déjà identifiés dans le produit obtenu selon la méthode 1 et montre que le mélange contenait encore 25% en poids de N-(1-hydroxy-3-phénylbutyl)anthranilate de méthyle (D).

Méthode 3

Dans un réacteur de 3 l, équipé d'un agitateur, d'une colonne Vigreux de 10 cm, d'un pont de distillation et d'un séparateur de fractions, on a chargé 815,4 g (5,4 mole) d'anthranilate de méthyle auquel on a ajouté, en 1h30, 666,0 g (4,5 mole) de 3-phénylbutanal. La température du réacteur est montée de 20° à 47-50°. On a continué l'agitation et monté progresssivement la température du mélange de la réaction à 65°. On a ensuite réduit la pression et, toujours sous agitation, on a augmenté la température du chauffage à 90-95° en 6 h, pendant que l'on montait progressivement la pression à $10^2$ Pa. On a recueilli 90 g de distillat et obtenu le produit désiré comme résidu (1385 g; rend. 93,5%).
L'analyse RMN de ce produit montrait qu'il s'agissait d'un mélange contenant environ 69% en poids de D (voir méthode 2), 10% en poids de B, 10% en poids de C et 4% en poids de A. Il contenait également 7% en poids de l'aldéhyde de départ.

Les données analytiques RMN des composants individuels de ces mélanges étaient les suivantes :

A. N-(3-phénylbutylidène)anthranilate de méthyle

RMN($^1$H,360MHz,$CDCl_3$) : 1,34(d,J~7Hz,3H) ; 2,78(m,2H) ; 3,25(dxq,J~7, 7Hz, 1H) ; 7,58(J~5Hz,1H) $\delta$ ppm

B. (E)-N-(3-phényl-1-buténylanthranilate de méthyle

RMN($^1$H,360MHz,$CDCl_3$) : 1,41(d,J~7Hz,3H) ; 3,53(quint,J~7Hz,1H) ; 3,83(s,3H) ; 5,34(dd,J~13, 7Hz,1H) ; 6,50(dd,J~13, 10Hz,1H) ; 6,63-7,83(plusieurs m,9H) ; 9,57(large d,J~10Hz,1H) $\delta$ ppm

RMN($^{13}$C,$CDCl_3$) : 22,4(q) ; 40,3(d) ; 51,7(s) ; 115,6(d) ; 124,6(d) $\delta$ ppm

C. (Z)-N-(3-phényl-1-butényl)anthranilate de méthyle

RMN($^1$H,360MHz,$CDCl_3$) : 1,44(d,J~7H,3H) ; 3,86(m, part. couvert,1H) ; 3,86(s,3H) ; 4,81(t,J=9Hz,1H) ; 6,47(dd,J~11, 9Hz,1H) ; 6,63-7,83(plusieurs m,9H) ; 9,81(large d,J~11Hz,1H) $\delta$ ppm

RMN($^{13}$C,$CDCl_3$) : 22,5(q) ; 36,6(d) ; 51,7(q) ; 113,8(d) ; 122,6(d) $\delta$ ppm

D. N-(1-hydroxy-3-phénylbutyl)anthranilate de méthyle

RMN($^1$H,360MHz,$CDCl_3$) : 1,35(d,J=7Hz,3H) ; 2,06-2,24(m,2H) ; 3,05(m,1H) ; 3,80(s,3H) ; 4,71(m,1H) ; 6,20(d,J~8Hz,1H) ; 6,42(d,J~8Hz,1H) ; 6,54-8,00(plusieurs m,7H) ; 7,84-(m,part. couvert,1H) ; 8,11(d,J~6Hz,1H) $\delta$ ppm

RMN($^{13}$C,$CDCl_3$) : 22,4(q) ; 36,7(d) ; 44,8(t) ; 51,5(q) ; 61,4(d) $\delta$ ppm

L'invention sera maintenant décrite en détail à l'aide des exemples suivants.

Exemple 1

Préparation d'une composition parfumante

On a préparé une composition parfumante de base par mélange des ingrédients suivants :

| Ingrédients | Parties en poids |
|---|---|
| Aldéhyde hexylcinnamique | 2000 |
| Salicylate de benzyle | 2000 |
| Linalol | 1000 |
| Citronellol | 1000 |
| Glycolate d'allyl amyle à 10%* | 500 |
| Cyclohexylpropionate d'allyle | 100 |
| α-Ionone | 100 |
| Essence de cassis synth. | 200 |
| Essence de jasmin synth. | 500 |
| 2,4-Diméthyl-3-cyclohexène-1-carbaldéhyde | 300 |
| EXALTEX ® [1)] | 800 |
| FLOROL ® [2)] | 500 |
| Total | 9500 |

* dans le dipropylèneglycol
1) mélange contenant la cyclopentadécanolide ; origine : Firmenich SA, Genève, Suisse
2) tétrahydro-2-isobutyl-4-méthyl-4(2H)-pyranol ; origine : Firmenich SA, Genève, Suisse

A cette composition de base de type floral-vert, on a ajouté 250 parties en poids du produit de réaction selon l'invention. On a ainsi obtenu une nouvelle composition dont la note odorante avait un net caractère floral, évoquant l'odeur des fleurs jaunes (genre bouton d'or). Cette composition avait une odeur complètement différente de celle que l'on obtient lors d'un usage des bases de Schiff connues dans les mêmes conditions, le caractère odorant étant totalement dans une direction de la note florale très naturelle et pas du tout dans celle de la fleur d'oranger.

Exemple 2

Test de substantivité

Un lot de textiles a été traité, pendant le cycle de lavage, avec un adoucissant textile auquel on avait ajouté 0,1% en poids du produit de réaction selon l'invention. Le linge humide exhalait, après ce traitement, une odeur caractérisée par une note plaisante de type fleur jaune. Quant au linge sec, il dégageait toujours une forte odeur florale-verte, le côté vert rappelant le caractère odorant du 3-phénylbutanal. La substantivité ainsi constatée du produit de l'invention se révèle d'autant plus surprenante et intéressante que l'aldéhyde de départ susmentionné n'est pas du tout substantif.

**Revendications**

**1.** Produit obtenu par la réaction de l'anthranilate de méthyle avec le 3-phénylbutanal, contenant au moins un composé de formule

(Ia)

ou

(Ib)

dans laquelle la ligne ondulée représente une liaison de conformation cis ou trans, ou encore de formule

(Ic)

2. Utilisation du produit selon la revendication 1 à titre d'ingrédient parfumant pour la préparation de compositions parfumantes ou d'articles parfumés.

3. Composition parfumante ou article parfumé résultant de l'utilisation selon la revendication 2.

4. A titre d'article parfumé selon la revendication 3, un parfum ou eau de toilette, un savon, un gel de douche ou de bain, un shampoing, une préparation cosmétique, un désodorisant corporel ou d'air ambiant, un détergent ou adoucissant textile ou un produit d'entretien.

5. Composition parfumante ou article parfumé selon la revendication 3, à odeur florale de type fleur jaune.

6. N-(3-Phényl-1-buténypanthranilate de méthyle.

7. N-(3-Phénylbutylidène)anthranilate de méthyle.

8. N-(1-Hydroxy-3-phénylbutyl)anthranilate de méthyle.

**Claims**

1. Product obtained by reacting methyl anthranilate with 3-phenylbutanal, containing at least one compound of formula

(Ia)

or

(Ib)

wherein the wavy line stands for a cis or trans configuration bond, or of formula

(Ic)

2. Use of a product according to claim 1 as a perfuming ingredient for the preparation of perfuming compositions or of perfumed articles.

3. Perfuming composition or perfumed article resulting from the use according to claim 2.

4. As a perfumed article according to claim 3, a perfume or cologne, a soap, a shower or bath gel, a shampoo, a cosmetic preparation, a body or air deodorant, a detergent or a fabric softener, or a household product.

5. Perfuming composition or perfumed article according to claim 3, having a floral odor of the yellow flower type.

6. Methyl N-(3-phenyl-1-butenyl)anthranilate.

7. Methyl N-(3-phenylbutylidene)anthranilate.

8. Methyl N-(1-hydroxy-3-phenylbutyl)anthranilate.

**Patentansprüche**

1. Produkt, erhalten durch Umsetzung von Methylanthranilat mit 3-Phenylbutanal, enthaltend zumindest eine Verbindung der Formel

(Ia)

oder

(Ib)

worin die Wellenlinie eine Bindung mit cis- oder trans-Konfiguration darstellt, oder auch der Formel

(Ic)

2. Verwendung des Produktes gemäss Patentanspruch 1 als Riechstoffbestandteil zur Herstellung von Riechstoffkompositionen oder von parfümierten Artikeln.

3. Riechstoffkomposition oder parfümierter Artikel, resultierend aus der Verwendung gemäss Patentanspruch 2.

4. Als parfümierter Artikel gemäss Patentanspruch 3, ein Parfüm oder ein Toilettwasser, eine Seife, ein Douche- oder ein Badegel, ein Shampoo, eine kosmetische Zubereitung, ein Körperdesodorant oder ein Raumluftverbesserer, ein Detergenz oder ein Textilweichmacher oder ein Pflegeprodukt.

5. Riechstoffkomposition oder parfümierter Artikel gemäss Patentanspruch 3 mit blumigem Duft vom Typus gelbe Blume.

6. N-(3-Phenyl-1-butenyl)methylanthranilat.

7. N-(3-Phenylbutyliden)methylanthranilat.

8. N-(1-Hydroxy-3-phenylbutyl)methylanthranilat.